(19) Europäisches Patentamt

European Patent Office

Office européen des brevets

(11) **EP 3 302 236 B1**

(12) **EUROPEAN PATENT SPECIFICATION**

(45) Date of publication and mention of the grant of the patent:
**01.04.2020 Bulletin 2020/14**

(21) Application number: **16730142.3**

(22) Date of filing: **02.06.2016**

(51) Int Cl.:
***A61B 5/00*** *(2006.01)*

(86) International application number:
**PCT/US2016/035467**

(87) International publication number:
**WO 2016/196761 (08.12.2016 Gazette 2016/49)**

(54) **SYSTEM FOR PAIN ASSESMENT**

SYSTEM FÜR SCHMERZBEURTEILUNG

SYSTÈME D'ÉVALUATION DE DOULEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2015 US 201562170246 P**

(43) Date of publication of application:
**11.04.2018 Bulletin 2018/15**

(73) Proprietor: **Boston Scientific Neuromodulation Corporation**
**Valencia, CA 91355 (US)**

(72) Inventors:
• **SHETAKE, Jai**
**Santa Clarita, California 91354 (US)**

• **LIN, Sherry**
**Glendale, California 91208 (US)**
• **MEKEL-BOBROV, Nitzan**
**Malibu, California 90265 (US)**

(74) Representative: **Vossius & Partner**
**Patentanwälte Rechtsanwälte mbB**
**Siebertstrasse 3**
**81675 München (DE)**

(56) References cited:
**WO-A1-2015/036661        US-A1- 2001 037 222**
**US-A1- 2013 018 275**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CLAIM OF PRIORITY

This application claims the benefit of priority of U.S. Provisional Patent Application Serial Number 62/170,246, filed on June 3, 2015, which is herein incorporated by reference in its entirety.

TECHNICAL FIELD

This document relates generally to medical systems, and more particularly, to systems and methods for assessing pain in a subject.

BACKGROUND

Pain is one of the most common and among the most personally compelling reasons for seeking medical attention, and consumes considerable healthcare resources each year. The relation between etiology, underlying mechanisms and the specific symptoms and signs related to painful disorders is complex. Pain in an individual patient may be produced by more than one mechanism.

Chronic pain, such as pain present most of the time for a period of six months or longer during the prior year, is a highly pervasive complaint and consistently associated with psychological illness. Chronic pain may originate with a trauma, injury or infection, or there may be an ongoing cause of pain. Chronic pain may also present in the absence of any past injury or evidence of body damage. Common chronic pain can include headache, low back pain, cancer pain, arthritis pain, neurogenic pain (pain resulting from damage to the peripheral nerves or to the central nervous system), psychogenic pain (pain not due to past disease or injury or any visible sign of damage inside or outside the nervous system).

In patients with chronic pain, multi-site pain, such as chronic musculoskeletal pain in multiple body sites, can affect more people than single-site chronic pain, and have more significant impact on a patient's quality of life, health care utilization and mental health. Due to differences of pain intensity and pain quality in various areas in the body, characterization of multi-site pain can be more complex than characterization of single-site pain.

Various agents or methods can be used to treat or to alleviate chronic pain, including medications, acupuncture, surgery, and neuromodulation therapy such as local electrical stimulation or brain stimulation, among others. A neuromodulation system can electrically stimulate tissue or nerve centers to treat nervous or muscular disorders. One class of neuromodulation systems include a spinal cord stimulation (SCS) system that can deliver electrical pulses to a specified region of a patient's spinal cord, such as particular spinal nerve roots or nerve bundles, to create an analgesic effect that masks pain sensation.

US-2001/037222-A1, WO-2015/036661-A1 and US-2013/018275-A1 show known systems for assessing pain.

OVERVIEW

By way of example, managing patients with chronic pain may involve determining appropriate treatment regimens such as SCS and evaluating the effectiveness of a particular therapy. Thus, it is desirable to efficiently assess and characterize pain. Current strategies for assessing pain severity, or the efficacy of a given pain therapy, generally rely on global outcome measures. For example, patients may be asked to rate the overall intensity of their pain on categorical scales ranging from no pain to severe pain. Also widely used are numerical rating scales of sensed pain intensity, such as a numeric rating scale (NRS) or a visual analog scale (VAS). The NRS allows a patients to provide a numeric rating for their pain intensity on a scale of 1 to 10, where 10 stands for the worst possible pain, and 1 stands for the minimal level of pain. The VAS, similar to the NRS, is an analog rating scale where the pain intensity is indicated on a horizontal line or similar depiction scaled from 0 to 100. Another pain assessment tool is a pain drawing, which can include anterior and posterior body diagrams on which a patient can draw or identify the anatomical distribution on the body of the pain that he/she suffers. The pain drawing can be used to assess percent area in pain, number of painful areas, or other characteristics.

Multi-site pain is a highly significant clinical problem among chronic pain patients. Characterization of multi-site pain may be desirable for pain therapies such as SCS, where many separate pain areas may be targeted. However, the methods as discussed above, although commonly used, have been inadequate at characterizing multi-site pain. For example, using the NRS or VAS requires the patient to associate only one number for their pain. This can be problematic for a couple of reasons. First, these scales can be subjective in nature, with ratings depending on the subjective past experiences of every patient. Second, associating one pain intensity number is not appropriate for multi-site pain patients, with patients reporting pain intensity of only one most bothersome area, or an aggregate of all painful areas. Therefore, using these global scales can result in inaccurate or inadequate pain characterization, and has implications on the interpretation of clinical outcomes in pain patients. Pain drawings, may be able to capture more detailed information than the VAS or NRS scores, they may require multiple quantifiers to describe pain in every patient, which can be cumbersome. Furthermore, each individual pain quantifier provides only partial information about the pain. For example, knowledge of the number of painful areas does not provide an insight into the spatial extent of the pain areas, and vice versa. Also none of the commonly used methods capture other relevant information about pain, such as the pain persistence or pain quality. Therefore, the present inventors have recognized, among other things, that there remains a demand for methods that combine several pain quantifiers in a unified manner, so as to provide a complete, efficient, and robust characterization of multi-site pain.

The invention is defined by the claims.

This document discusses, among other things, a system for assessing pain in a subject. The system can receive pain data, such as a patient pain drawing and a dermatome map, and generate pain descriptors using the pain data. The pain descriptors can be used to produce a composite pain score to quantify the subject's pain. The system can output diagnostic information including the pain score or the pain descriptors.

Example 1 can include subject matter (such as a device, apparatus, or machine) that comprises a data receiver, a processor circuit, and an output unit. The data receiver can be configured to receive pain data indicative of pain in the subject. The processor circuit can be configured to generate a plurality of pain descriptors using the pain data. The plurality of pain descriptors can be indicative of two or more of pain intensity, spatial extent of pain, or temporal pattern of pain. The processor circuit can produce a composite pain score that quantifies overall pain using the plurality of the pain descriptors. The output unit can be configured to produce a presentation of information about the pain in the subject, including the composite pain score.

Example 2 can include, or can optionally be combined with the subject matter of Example 1 to optionally include, the composite pain score that can be indicative of pain in the subject after delivery of a pain therapy. The output unit can be configured to produce the presentation including a pain therapy efficacy indicator based on a difference between the composite pain score and a reference pain score indicative of pain in the subject prior to delivery of the pain therapy.

Example 3 can include, or can optionally be combined with the subject matter of one or any combination of Examples 1 or 2 to include, the data receiver that can be configured to receive the pain data including one or more of a patient pain drawing, a patient questionnaire, or a dermatome map.

Example 4 can include, or can optionally be combined with the subject matter of one or any combination of Examples 1 through 3 to include, a user interface unit that can be communicatively coupled to the data receiver, and configured to enable the subject to input the pain data.

Example 5 can include, or can optionally be combined with the subject matter of one or any combination of Examples 1 through 4 to include, an image processing circuit, such as included within the processor circuit, that can be configured to produce a pixelated pain representation using the received pain data, including pixelating the patient pain drawing into a plurality of pixels corresponding to anatomical point locations of the subject, each of the plurality of pixels associated with respective pain descriptors indicating one or more of presence, intensity, or temporal pattern of pain at the corresponding pixel. The image processing circuit can group the pixels into one or more pain areas, each of the pain areas comprising pixels with respective pain descriptors meeting a specified criterion.

Example 6 can include, or can optionally be combined with the subject matter of Example 5 to optionally include, the processor circuit that can be configured to generate, for each of the pain areas, a respective plurality of pain descriptors including two or more of: a size of pain area indicating a proportion of a body of the subject affected by the respective pain area; an average pain intensity in the respective pain area; a peak pain intensity in the respective pain area; a pain persistence level in the respective pain area; or one or more dermatome numbers indicating vertebral levels of the subject affected by the respective pain area.

Example 7 can include, or can optionally be combined with the subject matter of Example 6 to optionally include, the one or more dermatome numbers that can be represented by integers each mapped to the vertebral levels associated with dermatomes C2 to S5. The dermatome numbers can include a maximum dermatome number and a minimum dermatome number associated with the respective pain area.

Example 8 can include, or can optionally be combined with the subject matter of Example 6 to include, the processor circuit that can be configured to generate one or more subject-level pain descriptors using the respective plurality of pain descriptors for the pain areas. The subject-level pain descriptors can include one or more of: an accumulative pain intensity across at least some of the pain areas; an accumulative pain persistence level across at least some of the pain areas; an anatomical pain span indicating a range of dematomal regions to which the pain areas extend; or a total number of pain areas.

Example 9 can include, or can optionally be combined with the subject matter of Example 8 to include, the accumulative pain intensity that can include a combination, across the pain areas, of one or both of the peak pain intensity and the average pain intensity in the respective pain area weighted by the size of the respective pain area.

Example 10 can include, or can optionally be combined with the subject matter of Example 8 to include, the accumulative pain persistence level that can include a combination, across the pain areas, of the pain persistence level in the respective pain area weighted by the size of the respective pain area.

Example 11 can include, or can optionally be combined with the subject matter of Example 8 to include, the anatomical pain span that can include a difference between a maximum dermatome number across the pain areas and a minimum dermatome number across the pain areas.

Example 12 can include, or can optionally be combined with the subject matter of Example 8 to include, the processor circuit that can be configured to produce the composite pain score using a linear or nonlinear combination of the subject-level pain descriptors.

Example 13 can include, or can optionally be combined with the subject matter of Example 12 to include, the processor circuit that can be configured to produce the composite pain score using a weighted sum of the subject-level pain descriptors.

Example 14 can include, or can optionally be combined with the subject matter of Example 12 to include, the processor circuit that can be configured to perform a transformation of the composite pain score.

Example 15 can include, or can optionally be combined with the subject matter of Example 12 to include, the processor circuit that can be configured to produce a normalized composite pain score by normalizing the composite pain score with respect to an expected range of the composite pain score.

Example 16 can include a machine-readable storage medium that can include instructions that, when executed by a machine, cause the machine to receive pain data indicative of pain in a subject, and use the pain data to generate a plurality of pain descriptors indicative of two or more of pain intensity, spatial extent of pain, or temporal pattern of pain. The instructions can further cause the machine to produce a composite pain score that quantifies overall pain using the plurality of the pain descriptors, and to produce a presentation of information about the pain in the subject for displaying in an output unit, the information including the composite pain score.

Example 17 can include a method for assessing pain in a subject. The method can comprise steps of receiving pain data indicative of pain in the subject, using the pain data to generate a plurality of pain descriptors that can be indicative of two or more of pain intensity, spatial extent of pain, or temporal pattern of pain, producing a composite pain score that quantifies overall pain using the plurality of the pain descriptors, and producing a presentation of information about the pain in the subject including the composite pain score.

Example 18 can include, or can optionally be combined with the subject matter of Example 17, to optionally include an operation of producing a pain therapy efficacy indicator using a comparison between the composite pain score and a reference pain score. The composite pain score can be indicative of overall pain in the subject after delivery of the pain therapy, and the reference pain score is indicative of overall pain in the subject prior to delivery of the pain therapy.

Example 19 can include, or can optionally be combined with the subject matter of Example 17 to optionally include, receiving data from one or more of a patient pain drawing, a patient questionnaire, or a dermatome map.

Example 20 can include, or can optionally be combined with the subject matter of Example 17 to optionally include steps of producing a pixelated pain representation using the received pain data, including pixelating the patient pain drawing into a plurality of pixels corresponding to anatomical point locations of the subject, each of the plurality of pixels associated with respective pain descriptors indicating one or more of presence, intensity, or temporal pattern of pain at the corresponding pixel, and grouping the pixels into one or more pain areas, each of the pain areas comprising pixels with respective pain descriptors meeting a specified criterion.

Example 21 can include, or can optionally be combined with the subject matter of Example 20 to optionally include, generating the pain descriptors which includes generating, for each of the pain areas, a respective plurality of pain descriptors including two or more of: a size of pain area indicating a proportion of a body of the subject affected by the respective pain area; an average pain intensity in the respective pain area; a peak pain intensity in the respective pain area; a pain persistence level in the respective pain area; or one or more dermatome numbers indicating vertebral levels of the subject affected by the respective pain area.

Example 22 can include, or can optionally be combined with the subject matter of Example 20 to optionally include, producing the composite pain score, which can include generating one or more subject-level pain descriptors using the respective pain descriptors for the pain areas, and producing the composite pain score using a linear or nonlinear combination of the subject-level pain descriptors. The subject-level pain descriptors can include one or more of: an accumulative pain intensity across at least some of the pain areas; an accumulative pain persistence level across at least some of the pain areas; an anatomical pain span indicating a range of dermatomal regions to which the pain areas extend, the anatomical pain span including a difference between a maximum dermatome number across the pain areas and a minimum dermatome number across the pain areas; or a total number of pain areas.

Example 23 can include, or can optionally be combined with the subject matter of Example 17, to optionally include, producing a logarithm transformation of the composite pain score, and normalizing the transformed composite pain score with respect to an expected range of the transformed composite pain score.

This Overview is an overview of some of the teachings of the present application and not intended to be an exclusive or exhaustive treatment of the present subject matter. Further details about the present subject matter are found in the detailed description and appended claims. Other aspects of the invention will be apparent to persons skilled in the art upon reading and understanding the following detailed description and viewing the drawings that form a part thereof, each of which are not to be taken in a limiting sense. The scope of the present invention is defined by the appended claims and their legal equivalents.

BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.

FIG. 1 illustrates generally an example of a pain management system and portions of an environment in which the system can operate.
FIG. 2 illustrates generally an example of a pain assessment circuit.
FIG. 3 illustrates generally an example of dermatomes in a human body and the corresponding spinal nerves coming from the spinal cord.
FIG. 4 illustrates generally an example of a mapping between the dermatomes and dermatome numbers.
FIG. 5 illustrates generally an example of a pain descriptor generator.
FIG. 6 illustrates generally an example of a composite score generator.
FIG. 7 illustrates generally an example of a method for assessing pain in a subject.
FIG. 8 illustrates generally an example of a method for generating pain descriptors.
FIG. 9 illustrates generally an example of a method for producing a composite pain score.

DETAILED DESCRIPTION

Disclosed herein are systems, devices, and methods for assessing pain in a subject. The system can receive pain data, such as a patient pain drawing and a dermatome map, and produce a composite pain score that quantifies the subject's pain. The composite pain score, such as an integrated pain quantification index (IPQI), can be used throughout the pain treatment of a patient. For example, the IPQI can be evaluated during patient follow-ups to demonstrate efficacy of a prescribed pain therapy, at least based on changes of the IPQI before and after the pain treatment. The IPQI can also be used by the clinician to optimize treatment and patient management, such as by comparing benefits of different therapy regimens including, for example, different electrode configurations or therapy parameters associated with SCS. In addition to the intra-subject pain assessment, the IPQI can also be used for inter-patient pain assessment, such as by identifying, among a cohort of patients having different pain intensity, anatomical pain extent, or persistence, those patients that have relatively more severe pain who require more intensive treatment.

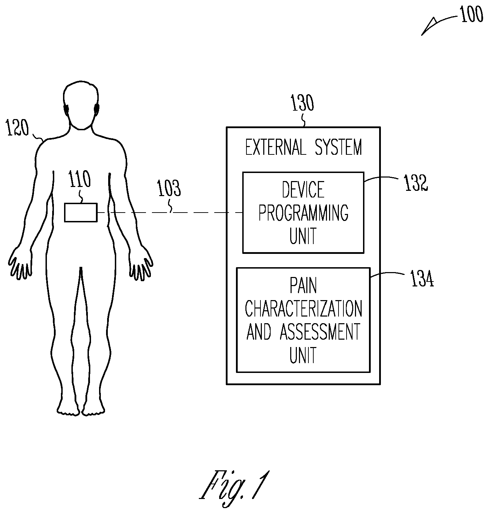

FIG. 1 illustrates generally an example of a pain management system 100 and portions of an environment in which the pain management system 100 can operate. The pain management system 100 can include an ambulatory medical device, such as an implantable medical device (IMD) 110, that is associated with a subject 120 such as a patient suffering from chronic pain. The IMD 110 can be in a form of a subcutaneously implanted device, a wearable external device, a neural stimulator, a drug delivery device, a biological therapy device, a diagnostic device, or one or more of other ambulatory medical devices. The IMD 110 can be configured to deliver therapy to one or more body sites of the subject 120 for treating chronic pain. In an example, the pain therapy can include neuromodulation therapy, and the IMD 110 can deliver focused (rather than systemic throughout the body such as pharmaceutical treatments) delivery of modifying agents to targeted areas of the nervous system in order to improve neural function. The modifying agents can include pharmaceutical agents, electrical energy, magnetic energy, or other forms of energy.

In an example, the IMD 100 can include a pulse generator that can generate electrostimulation for stimulating local tissues, such as one or more specified regions of a patient's spinal cord, such as particular spinal nerve roots or nerve bundles. Such spinal cord stimulation (SCS), when applied to the spinal cord associated with regions of the body afflicted with chronic pain, can induce paresthesia, or a subjective sensation of numbness or tingling, in the afflicted bodily regions. This paresthesia can effectively mask the transmission of non-acute pain sensations to the brain.

The IMD 100 can include a hermetically sealed can that can house circuitry for generating the electrical pulses, control circuitry, communication circuitry, a battery, etc. The IMD 100 can be electrically coupled to the stimulation sites such as through one or more leads. Each lead can include a longitudinal lead body comprising insulative material coating that encloses wire conductors. Along the lead body, or at the distal end of the lead body, includes one or more electrodes that are electrically coupled to the wire conductors. The proximal end of the lead body can include one or more terminals that can be electrically coupled to the wire conductors and receive electrostimulation pulses. The distal end of a respective stimulation lead can be implanted within the epidural space to deliver the electrical pulses to the appropriate nerve tissue at the specified stimulation sites within the spinal cord.

The pain management system 100 can include an external system 130 that can communicate with the IMD 110 such as via a communication link 103. The external system 130 can allow for programming of the IMD 110, and can receive information about one or more signals acquired by IMD 110. The communication link 103 can include one or more of an inductive telemetry link, a radio-frequency telemetry link, or a telecommunication link, such as an internet connection. The communication link 103 can provide for data transmission between the IMD 110 and the external system 130. The transmitted data can include, for example, real-time physiological data acquired by the IMD 110, physiological data acquired by and stored in the IMD 110, therapy history data or data indicating IMD operational status stored in the IMD 110, one or more programming instructions to the IMD 110 such as to configure the IMD 110 to perform one or more actions that can include physiological data acquisition such as using programmably specifiable sensing electrodes and configuration, device self-diagnostic test, or delivery of one or more therapies.

As illustrated in FIG. 1, the external system 130 can include a device programming unit 132. The device programming unit 132 can be used, such as by a clinician, to program the parameters or features in the IMD 110. Additionally or alternatively, the device programming unit 132 can include an input device that enables the subject 120 to adjust the therapy parameters within a predetermined range to improve therapy outcome. Alternatively or additionally, the external system 130 can include a remote patient management system that can monitor patient status or adjust one or more therapies such as from a remote location.

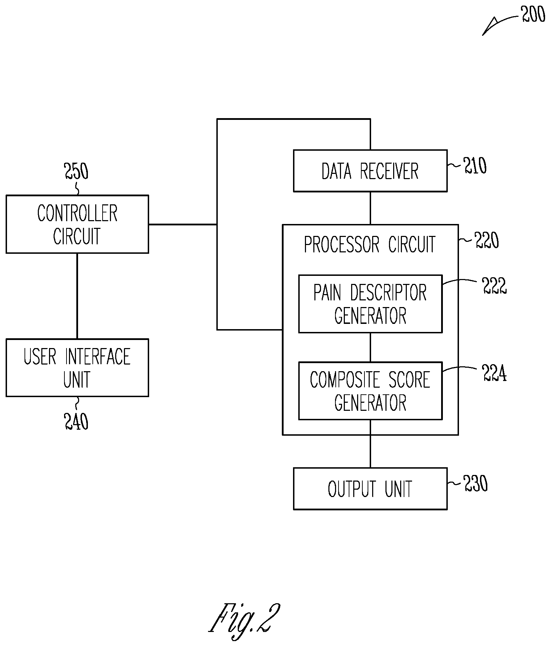

The external system 130 can comprise a pain characterization and assessment unit 134, which can receive a multitude of pain descriptors from various sources and produce a synthesized indicator that objectively quantifies intensity, persistence, and extensiveness of pain suffered by the subject 120. The pain indicators can be used to assess efficacy of pain therapy. In an example, the pain characterization and assessment unit 134 can be implemented as machine-readable storage medium, such as software, including instructions executable by a machine to provide human-perceptible diagnostic information about pain. In an example, at least a part of the machine-readable storage medium can be incorporated into the device programming unit 132. The device programming unit 132 can prompt a clinician to adjust the pain therapy, at least by using the pain diagnostic information such as provided by the pain characterization and assessment unit 134. Examples of the pain characterization and assessment unit 134 are described below, such as with reference to FIGS. 2-6.

Portions of the IMD 110 or the external system 130 can be implemented using hardware, software, firmware or combinations of hardware thereof. Portions of the IMD 110 or the external system 130 may be implemented using an application-specific circuit that can be constructed or configured to perform one or more particular functions, or can be implemented using a general-purpose circuit that can be programmed or otherwise configured to perform one or more particular functions. Such a general-purpose circuit can include a microprocessor or a portion thereof, a microcontroller or a portion thereof, or a programmable logic circuit, or a portion thereof. For example, a "comparator" can include, among other things, an electronic circuit comparator that can be constructed to perform the specific function of a comparison between two signals or the comparator can be implemented as a portion of a general-purpose circuit that can be driven by a code instructing a portion of the general-purpose circuit to perform a comparison between the two signals. While described with reference to the IMD 110, the pain management system 100 could include a subcutaneous medical device, wearable medical devices, or other external medical devices.

FIG. 2 illustrates generally an example of a pain assessment circuit 200, which can be an embodiment of the pain characterization and assessment unit 134. The pain assessment circuit 200 can include one or more of an data receiver circuit 210, a processor circuit 220, an output unit 230, a user interface unit 240, and a controller circuit 250.

The data receiver circuit 210 can receive pain data indicative of pain in the subject. The pain data can be in one or more of textual, graphical, verbal, or other formats of representation. The pain data can include subjective pain description, such as a pain drawing or a patient questionnaire, produced by the subject. The pain drawings or the patient questionnaire can include information of anatomical locations and distribution of the pain, intensity of pain at various pain locations, or temporal pattern such as persistence of the pain at various pain locations. For example, the pain drawing can include illustrative diagrams of human body including one or both of ventral or dorsal depiction of the body. The subject may mark the diagrams with pain markings to identify the anatomical locations where pain radiates or expands. The pain markings can indicate the pain the subject presently senses, or suffers within a specified period of time, such as in past seven days prior to the time of marking. The pain markings can additionally include different symbols to distinguish various pain sensations, such as aching, numbness, burning, stabbing, or needle pain. Additionally or alternatively, the pain markings can include different symbols to distinguish different intensities of the pain at each marked pain location.

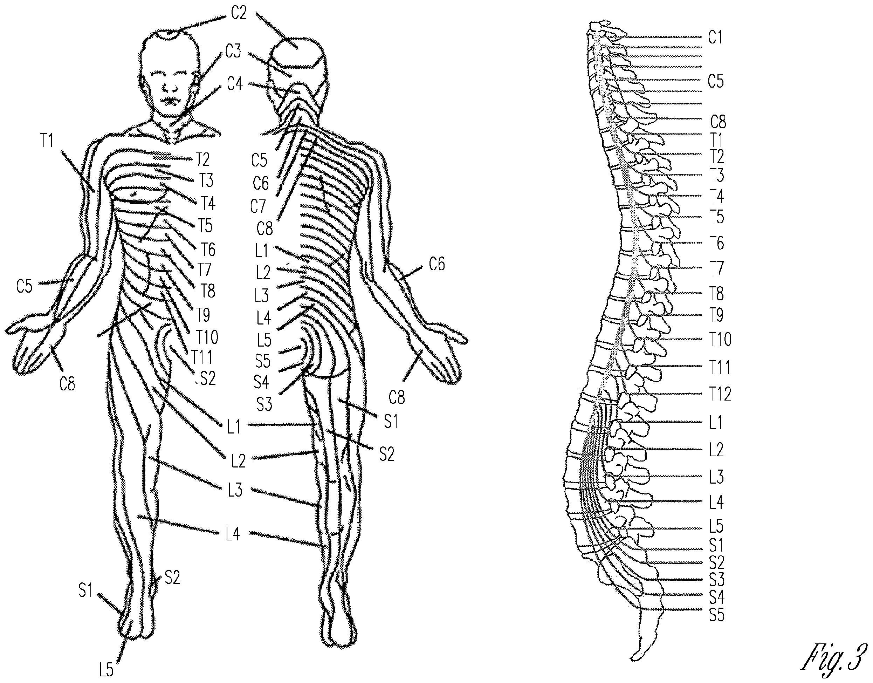

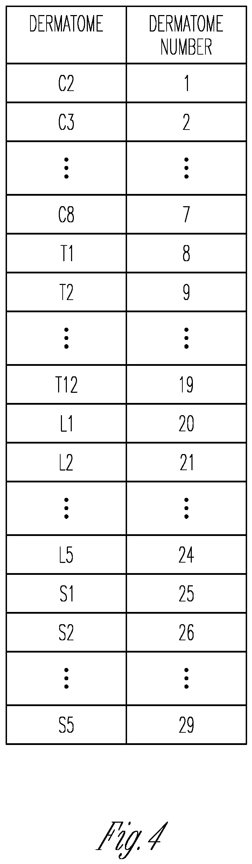

The pain data can also include reference data, such as a dermatome map. A dermatome map can comprise areas of skin each labeled with the spinal nerve innervating it. Examples of dermatomes and the corresponding dermatome numbers, which can be used for characterizing the spatial extent of pain, are described below, such as with reference to FIGS. 3 and 4.

The processor circuit 220 can be configured to produce a composite score, such as an integrated pain quantification index (IPQI), using the received pain data. The processor circuit 220 can be implemented as a part of a microprocessor circuit within the pain assessment circuit 200. The microprocessor circuit can be a dedicated processor such as a digital signal processor, application specific integrated circuit (ASIC), microprocessor, or other type of processor for processing information including physical activity information. Alternatively, the microprocessor circuit can be a general purpose processor that can receive and execute a set of instructions of performing the functions, methods, or techniques described herein.

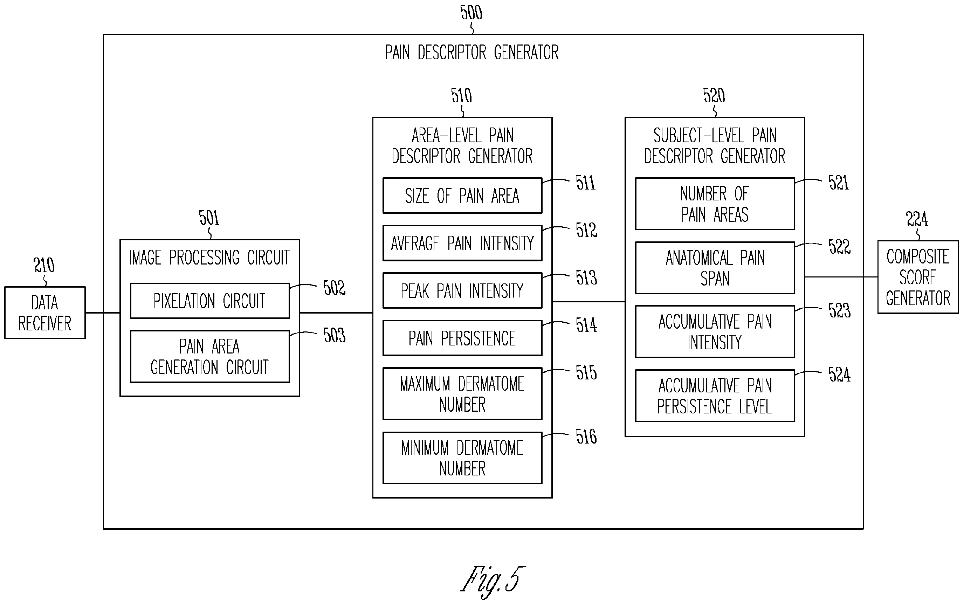

The processor circuit 220 can include a pain descriptor generator 222 and a composite score generator 224. The pain descriptor generator 222 can generate a plurality of pain descriptors using the pain data. The pain descriptors can be indicative of one or more of pain intensity, spatial extent of pain, or temporal pattern of pain. The pain intensity can be represented by a numerical or categorical value indicating the intensity of pain at a specified body site. The spatial extent of pain can be represented by a numerical or categorical value indicating the anatomical span of pain. The temporal pattern of pain can be represented by a numerical or categorical value indicating persistence of pain at a specified body site. The pain descriptors can additionally include pain quality indicators indicating sensations of pain. The pain descriptors can be automatically generated using the pain data such as the pain drawing provided by the subject, and quantitatively describe the pain in a pain-area level or in a subject-level. Examples of pain descriptor generator 222 are described below, such as with reference to FIG. 5.

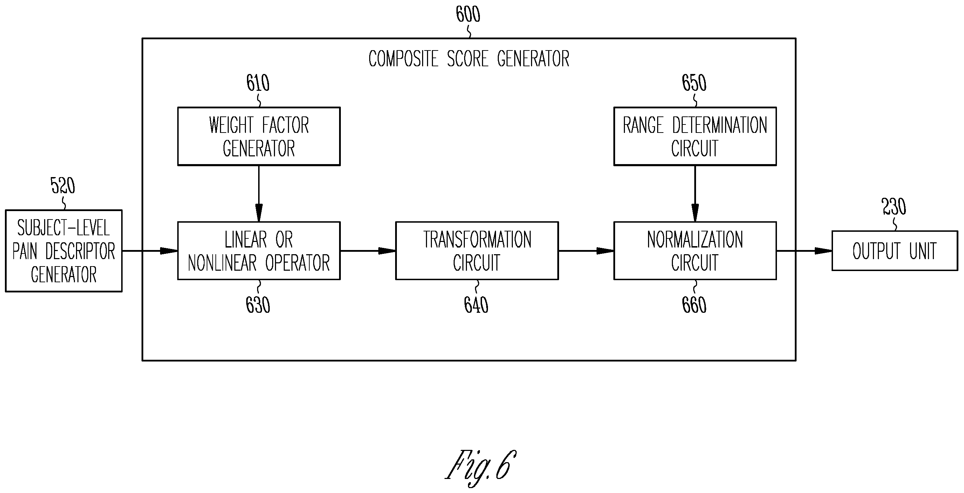

The composite score generator 224 can produce a composite pain score, such as an integrated pain quantification index (IPQI), using the plurality of the pain descriptors. The composite pain score can be represented as a numerical or categorical value that quantifies overall pain in the subject. In an example, the composite pain score can be a linear or nonlinear combination of the pain descriptors such as produced by the pain descriptor generator 222. Examples of pain descriptor 222 are described below, such as with reference to FIG. 6.

The output unit 230 can be configured to generate a presentation of information about the pain in the subject. The output unit 230 can include a monitor or other displaying devices configured to display the information about the pain in one or more human-perceptible medium formats. The displayed information can include the composite pain score, pain descriptors for one or more identified pain area, or pain descriptors in a subject-level that characterize overall pain intensity, pain persistence, or anatomical extent. The output unit 230 can additionally display other diagnostic information related to the pain or therapeutic information related to the treatment prescribed. In an example, the output unit 230 can be configured to present information indicating effectiveness, or patient response, to a pain therapy. For example, the processor circuit 220 can generate a reference pain score before delivery of a prescribed pain therapy, such as a SCS therapy with specified stimulation configuration and parameters. After delivery of the prescribed pain therapy, the processor circuit 220 can generate a composite pain score. The output unit 230 can produce the presentation including a pain therapy efficacy indicator based on a comparison between the composite pain score and the reference pain score. The pain therapy efficacy indicator can be reviewed by a clinician to assess the effectiveness of the prescribed therapy. If the pain therapy efficacy indicator does not meet a therapeutic criterion, the clinician can modify the pain therapy from the user interface unit 240, such as by reconfiguring the SCS stimulation electrodes, stimulation sites, or adjusting the SCS frequency, intensity, or duration.

The user interface unit 240 can include an input device, such as a keyboard, on-screen keyboard, mouse, trackball, touchpad, touch-screen, or other pointing or navigating devices. The input device can also include electronic interactive writing or graphic drawing devices, such as a tablet or a computer, that enable the subject to input the pain data. In an example, the input device can be used by the user to generate pain drawings or to answer a patient questionnaire. The input pain data received from the user interface unit 240 can be controllably transmitted to the data receiver 210 for use in pain assessment.

In some examples, the pain data such as pain drawings and patient questionnaire can be presented in a paper format, and the user interface unit 240 can enable a user to transfer the pain data to the pain assessment circuit 200 in a digitized format, such as by scanning and digitizing the pain drawings or encoding the answers to the patient questionnaire. In some examples, the pain data can be pre-generated and stored in a storage device such as an electronic medical record (EMR) system, and the data receiver 210 can retrieve the pain data from the storage device upon receiving a command signal provided by a system user.

The controller circuit 250 can control the operations of the data receiver 210, the processor circuit 220, the user input unit 240, and the data flow and instructions among these components and respective subcomponents. In an example, the controller circuit 250 can determine the input pain data to be received by the data receiver circuit 210. The controller circuit 250 can also control the generation of the pain descriptors and the composite scores.

FIG. 3 illustrates generally an example of dermatomes in a human body and the corresponding spinal nerves coming from the spinal cord. Originating from the spinal cord there are eight cervical nerves (C1 through C8), twelve thoracic nerves (T1 through T12), five lumbar nerves (L1 through L5), and five sacral nerves (S1 through S5). Each of these nerves relays sensations, including pain, from a particular region of skin to the brain. Successful pain management and the avoidance of stimulation in unafflicted regions require the applied electric field to be properly positioned longitudinally along the dorsal column

A dermatome is an area of skin that is supplied by sensory neurons that arise from a spinal nerve ganglion. Except for the cervical nerve C1, which maps to no dermatome, a total of 29 spinal nerves (including cervical nerves C2-C8, thoracic nerves T1-T12, lumbar nerves L1-L5, and sacral nerves S1-S5) can be mapped to a respective dermatome, resulting in 29 dermatomes distributed across the body surface. In particular, the head and neck regions are associated with C2 - C8, the back is associated with C2 -S3, the central diaphragm is associated with C3 - C5, the upper extremities are associated with C5 - T1, the thoracic wall is associated with T1 - T11, the peripheral diaphragm is associated with T6 - T11, the abdominal wall is associated with T6 -LI, lower extremities are located from L2 -S2, and the perineum from L4 -S4. For example, chronic pain sensations commonly focus on the lower back and lower extremities, which correspond to T8 - T10. A specific energy field can be applied to the corresponding spinal nerves to treat the chronic pain in the area.

FIG. 4 illustrates generally an example of a mapping between the dermatomes and dermatome numbers. The dermatome numbers, ranging from 1 to 29, correspond to the longitudinal arrangement of the spinal nerves C2 - S5 along the dorsal column. More superior spinal nerves are mapped to smaller dermatome numbers, and more inferior spinal nerves are mapped to larger dermatome numbers. The dermatome number can be used to characterize the spatial extent of pain. For example, the pain in a first patient affects dermatomes L3 to S2, which can be mapped to dermatome numbers 22 to 26; the pain in a second patient affects dermatomes T10 to S5, which can be mapped to dermatome numbers 17 to 29. The pain in the second patient (affecting 13 dermatomes) is said to have a greater spatial extend than the pain in the first patient (affecting only 5 dermatomes).

FIG. 5 illustrates generally an example of a pain descriptor generator 500, which can be an embodiment of the pain descriptor generator 222. The pain descriptor generator 500 can generate a plurality of pain descriptors using the pain data. The pain descriptor generator 500 can include one or more of an image processing circuit 501, an area-level descriptor generator 510, and a subj ect-level pain descriptor generator 520.

The image processing circuit 501 can be configured to automatically generate one or more pain areas based on the received pain data, such as provided by the data receiver 210. The image processing circuit 501 can include a pixelation circuit 502 and a pain area generation circuit 503. The pixelation circuit 502 can produce a pixelated pain representation using the received pain data. In an example, the pixelation circuit 502 can convert the patient pain drawings into a plurality of pixels represented by respective pixel coordinate corresponding to anatomical point locations of the subject. Each pixel can be associated with respective pain descriptors that indicate one or more of presence, intensity, or temporal pattern of pain at the corresponding pixel. In an example, each pixel can be associated with a value of "1" to indicate presence, or "0" to indicate absence, of pain sensation at that pixel location. In another example, if the receive pain data (such as the patient pain drawings) includes information about different pain sensations such as degree of pain intensity at various identified anatomical locations, each pixel can be associated with a number within a specified range, such as between 1 and 5, that indicates the intensity of pain at the pixel location. The scale may be an ascending scale where a larger number can correspond to a higher pain intensity. Alternatively, the scale may be a descending scale where a larger number can correspond to a lower pain intensity. In an example, each pixel can be associated with multi-dimensional features including presence, pain intensity, and pain persistence such as over a specified period of time, at the corresponding pixel location.

The pain area generation circuit 503 can use the pixels produced by the pixelation circuit 502 to generate $N$ pain areas, where $N$ is a positive integer. Each pain area can comprise pixels with respective pain descriptors meeting a specified criterion. In an example where the pixels are each associated with a binary value of "1" or "0" to respectively indicate the presence or absence of pain, the pain area generation circuit 503 can produce the $N$ pain areas by grouping the pixels having values of "1" and not substantially separated by pixels having values of "0". For example, pixels having value of "1" that are adjacent to each other or separated by no more than $K$ pixels having value of "0" can be grouped into one pain area, where $K$ is a positive integer. In an example, $K$ equals 1. In another example, the pain area generation circuit 503 can produce the N pain areas by performing clustering analysis of the coordinates of the pixels, where each pixel can be associated with multi-dimensional features such as pain presence, pain intensity, and pain persistence. Examples of the clustering analysis can include $k$-means clustering, which assigns the pixels into one or more pain areas (clusters), such that the within-cluster sum of squares of a similarity metric between the pixels within that cluster and a cluster centroid is minimized. A pixel can be classified as being in a pain area (or cluster) if the similarity metric meets a specified criterion, such as when the similarity metric falls within a specified range, below a specified threshold value, or being more similar (as measured by the similarity metric) to the assigned pain area than to other pain areas. When all the pixels are assigned to the pain areas, the pain areas can optionally be merged or further divided until the within-cluster sum of squares meets a convergence criterion. Examples of the similarity metric can include multi-dimensional statistical distance including an L1 norm, an L2 norm (i.e., Euclidian distance), an infinite norm or other norms in the normed vector space, a cosine distance or correlation coefficient, or Mahalanobis distance, among others.

The area-level pain descriptor generator 510 can generate, for each of the pain areas, a respective plurality of pain descriptors. Such pain area-level pain descriptors can include two or more of: a size of pain area 511, an average pain intensity 512, a peak pain intensity 513, a pain persistence level 514, or one or more dermatome numbers associated with the respective pain area such as a maximum dermatome number 515 and a minimum dermatome number 516. The size of a pain area 511, denoted by $X_j^{size}$, can indicate a proportion of a body of the subject affected by the pain area $j$. In an example, the pain size can be a percentage of pain coverage in dermatome area, ranging between 0 and 100%. The average pain intensity 512, denoted by $X_j^{aveIntensity}$, and the peak pain intensity 513, denoted by $X_j^{peakIntensity}$, can each be represented by a numerical value within a specified range indicating respectively average intensity and highest intensity of the pain at the pain area $j$ within a specified period of time, such as in the past 7-10 days. In an example, the average pain intensity 512 and the peak pain intensity 513 each can take a value between 0 and 3, where 0 indicates "no pain" and 3 indicates "worst imaginable degree of pain" in the respective pain area. The pain persistence level 514, denoted by $X_j^{persistence}$, can be represented by a numerical value within a specified range indicating frequency, duration, or continuity of the pain at the pain area $j$ within a specified period of time, such as in the past 7-10 days. In an example, the pain persistence level 514 can take value between 0 and 3, where 0 indicates "never" and 3 indicates "always" sensing the pain in the respective pain area. The maximum dermatome number 515, denoted by $X_j^{maxDerm}$, and a minimum dermatome number 516, $X_j^{minDerm}$, can indicate the anatomical extent of the pain at the pain area $j$, and can be determined by overlaying the dermatome map (such as illustrated in FIG. 3) with the identified pain areas based at least on the pain drawings. The dermatomes as covered by the respective pain area can be mapped to corresponding dermatome numbers, such as by using the mapping table illustrated in FIG. 4. For example, a pain area that covers skin areas corresponding to dermatomes T10 - S5 has a minimum dermatome number of 17 (corresponding to T10) and a maximum dermatome number of 29 (corresponding to S5), according to the mapping illustrated in FIG. 4.

The subject-level pain descriptor generator 520 can integrate the plurality of pain descriptors, such as the descriptors $X_j^{size}$, $X_j^{aveIntensity}$, $X_j^{peakIntensity}$, $X_j^{persistence}$, $X_j^{maxDerm}$, and $X_j^{minDerm}$, across all $N$ pain areas, as produced by the area-level pain descriptor generator 510, to produce one or more subject-level pain descriptors. The subject-level pain descriptors can include one or more of: a total number of pain areas ($N$) 521, an anatomical pain span 522, an accumulative pain intensity 523, and an accumulative pain persistence level 524 across at least some of the pain areas. The anatomical pain span 522, denoted by $Y^{span}$, can indicate a range of dematomal regions to which the pain area extend. In an example, the anatomical pain span can be computed as a difference between a maximum dermatome number across the $N$ pain areas and a minimum dermatome number across the $N$ pain areas, that is,

$$Y^{span} = \max\{X_j^{maxDerm}\}_{j=1,2,\ldots,N} - \min\{X_j^{minDerm}\}_{j=1,2,\ldots,N} \qquad (1)$$

The accumulative pain intensity 523, denoted by $Y^{SSI}$, can be computed as a combination such as a sum, across the $N$ pain areas, of a measure of pain intensity at the respective pain area $j$. The measure of pain intensity can computed using one or both of the peak pain intensity $X_j^{peakIntensity}$ and the average pain intensity $X_j^{aveIntensity}$. In an example, the measure of pain intensity at pain area $j$ can be computed as an average of $X_j^{peakIntensity}$ and $X_j^{aveIntensity}$, scaled by the size of the respective pain area $X_j^{size}$, that is,

$$Y^{SSI} = \sum_{j=1}^{N} X_j^{size} * (X_j^{aveIntensity} + X_j^{peakIntensity})/2 \qquad (2)$$

The accumulative pain persistence level 524, denoted by $Y^{SSP}$, can be computed as a combination such as a sum, across the $N$ pain areas, of the pain persistence level $X_j^{persistence}$ in the respective pain area $j$, scaled by the size of the respective pain area $X_j^{size}$, that is,

$$Y^{SSP} = \sum_{j=1}^{N} X_j^{size} * X_j^{persistence} \qquad (3)$$

The subject-level pain descriptors, including some or all of $Y^{span}$, $Y^{SSI}$, $Y^{SSP}$, and the total number of pain areas $N$, can be used by the composite score generator 224 to produce a composite pain score.

FIG. 6 illustrates generally an example of a composite score generator 600, which can be an embodiment of the composite score generator 224. The composite pain score can be computed as a linear or nonlinear combination of the subject-level pain descriptors. In an example as illustrated in FIG. 6, the composite score generator 600 can include a linear or nonlinear operator 630 that can produce the composite pain score, denoted by $Z^{raw}$, as a weighted sum of the subject-level pain descriptors 520 including $Y^{span}$, $Y^{SSI}$, $Y^{SSP}$, and N, that is,

$$Z^{raw} = w_1{*}N + w_2{*}Y^{span} + w_3{*}Y^{SSI} + w_4{*}Y^{SSP} \qquad (4)$$

The weight factors, $w_1$ through $w_4$, can be specified by a system user. Alternatively, the weight factor generator 610 can generate the weight factors based on principal component analysis (PCA) of a database of patient pain data. PCA is a mathematical procedure that can reduce a set of potentially correlated variables (*e.g.* pain distribution, pain intensity, and persistence) into a smaller set of variables that preserves most of the information. In an example, the PCA of the patient pain data can include standardizing each pain variable such as by subtracting off its mean and dividing by its standard deviation. A matrix of pain variables can be formed using the pain variables. Such matrix can then be factorized such as by using singular value decomposition (SVD). The right-singular vector, corresponding to the largest singular value, can be retained as the weight factors $w_1$ through $w_4$. As an example, the weight factors can be $w_1 = 0.6$, $w_2 = 0.4$, $w_3 = 0.5$, and $w_1 = 0.4$, suggesting that the number of painful areas N, which is multiplied by $w_1$ in equation (4), is the most influential in the calculation of the composite pain score $Z^{raw}$.

A transformation circuit 640 can transform the composite pain score to improve the interpretability of data. In an example, the transformation circuit 640 can perform logarithm transformation of the composite score, that is,

$$Z_T{}^{raw} = log\,(Z^{raw} + \delta) \qquad (5)$$

The logarithm transformation can improve the sensitivity of the composite pain score to changes in the subject-level pain descriptors when the patient has more localized or moderate amount of pain. The small constant $\delta$ included in Equation (5) can ensure that $Z_T{}^{raw}$ be bounded (i.e., $Z_T{}^{raw}$ would not take value of negative infinity when $Z^{raw}$ is zero or infinitesimally small).

The composite score generator 600 can include a normalization circuit 660 configured to produce a normalized composite pain score, such as an integrated pain quantification index (IPQI), by normalizing the composite pain score with respect to an expected range of the composite pain score, such as produced by the range determination circuit 650. In an example, the expected range can be determined as the difference between a theoretical maximum ($Z^{max}$) and a theoretical minimum ($Z^{min}$) amongst all possible composite pain scores. The theoretical minimum $Z^{min}$ can be obtained when no pain is sensed, corresponding to all subject-level pain descriptors $Y^{span}$, $Y^{SSI}$, $Y^{SSP}$, and $N$ being set to zero, that is, when $Z^{raw} = 0$. As a result,

$$Z^{min} = log\,(Z^{raw} + \delta) = log\,(\delta) \qquad (6)$$

The theoretical maximum $Z^{max}$ can be obtained when the subject senses full body, worst imaginable, and continuous pain, corresponding to all subject-level pain descriptors $Y^{span}$, $Y^{SSI}$, $Y^{SSP}$, and $N$ being set to maximum values within their respective ranges, that is,

$$Z^{max} = log\{w_1{*}\max(N) + w_2{*}\max(Y^{span}) + w_3{*}\max(Y^{SSI}) + w_4{*}\max(Y^{SSP}) + \delta\}$$

$$(7)$$

The normalized composite pain score, IPQI, can then be determined as:

$$IPQI = (Z_T{}^{raw} - Z^{min})\,/\,(Z^{max} - Z^{min}) \qquad (8)$$

The IPQI is a real number between 0 and 1, where IPQI = 0 corresponds to no pain, and IPQI = 1 corresponds to maximal pain $Z^{max}$. A presentation of pain information, including the IPQI, can then be passed to the output unit 230 for display.

In some example, the composite score generator 600 can perform a secondary pain assessment, including quantizing the IPQI further into one of two or more categorical pain zones. Each pain zone can be defined by a lower and a higher boundary IPQI value. For example, the IPQI can be quantized into one of four zones, including a first zone of "mild pain" such as defined by an IPQI range of (0, $IPQI_{T1}$), a second zone of "moderate pain" such as defined by an IPQI range of ($IPQI_{T1}$, $IPQI_{T2}$), a third zone of "major pain" defined by an IPQI range of ($IPQI_{T2}$, $IPQI_{T3}$), and a fourth zone of "severe pain" defined by an IPQI range of ($IPQI_{T3}$, 1). By way of example and not limitation, the boundary values may be $IPQI_{T1}$ = 0.3, $IPQI_{T2}$ = 0.5, $IPQI_{T3}$ = 0.7. In some examples, the IPQI can be combined with other indicators of quality of life, emotional function, physical activity, or neuro-functioning assessment to determine two or more categorical pain zones.

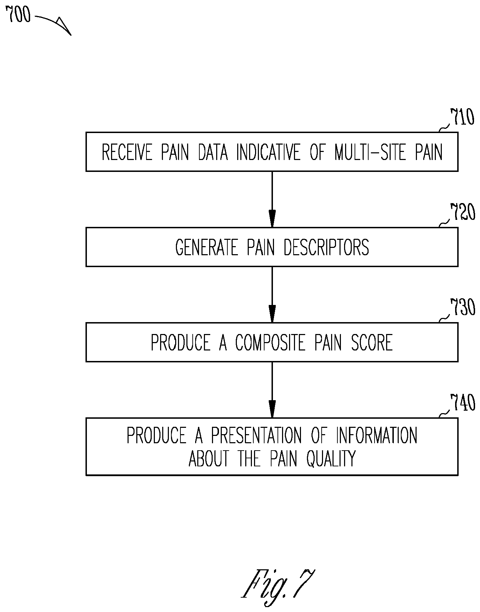

FIG. 7 illustrates generally an example of a method 700 for assessing pain in a subject. The method 700 can be implemented and operate in a medical system, such as a programmer or a remote server-based patient management system in communication with a ambulatory medical device configured to provide pain therapy to the patient. In an example, the method 700 can be performed by the pain assessment circuit 200, or any modification thereof.

In some examples, the method 700 can be used prior to implantation of a pain management device, such as a spinal cord stimulation (SCS) device, to provide assessment of the severity of pain. The assessment information can be used by a clinician to determine if the patient is a candidate for a particular pain management therapy, or to optimize device programming to patients indicated for such a therapy. The method 700 can additionally or alternatively be used after device implant or receiving pain therapy, such as during patient follow-ups. The pain assessment information can be used to evaluate progression of pain, or improvement of symptoms which demonstrates effectiveness of a prescribed pain therapy.

The method 700 can begin at step 710, where one or more sources of pain data can be received. The pain data can include subjective pain description, such as a pain drawing or a patient questionnaire, provided by the subject. The pain drawings or the patient questionnaire can include information of anatomical location and distribution of the pain, intensity of pain at various pain locations, or temporal pattern such as persistence of the pain at various pain locations. The pain drawing can include illustrative diagrams of human body, along with the pain markings provided by the subject that identify the anatomical locations where pain radiates or expands. The pain drawing can additionally include various symbols to distinguish different types sensations such as aching, numbness, burning, stabbing, or needle pain, among others, or different levels of intensity of the pain.

The pain data can also include a dermatome map. A dermatome map can comprise areas of skin each labeled with respective spinal nerve innervating it. A total of 29 spinal nerves, including cervical nerves C2-C8, thoracic nerves T1-T12, lumbar nerves L1-L5, and sacral nerves S1-S5, can each be mapped to a respective dermatome, such as according to the mapping illustrated in FIG. 4. Each dermatome can be assigned a unique dermatome number between 1 and 29, which corresponds to the longitudinal arrangement of the spinal nerves C2 - S5 along the dorsal column

In an example, some of the pain data, such as pain drawings and patient questionnaire, can be provided by the subject using a user interface unit comprising an input device such as a keyboard, on-screen keyboard, mouse, trackball, touchpad, touch screen, writing and graphic drawing tablets, among others. Alternatively, the pain data can be stored, and retrieved from a storage device such as an electronic medical record (EMR) system.

At 720, a plurality of pain descriptors can be generated using the received pain data. The pain descriptors can be indicative of two or more of pain intensity, spatial extent of pain, or temporal pattern of pain. The spatial extent of pain can be represented by a numerical or categorical value indicating the anatomical span of pain. The temporal pattern of pain can be represented by a numerical or categorical value indicating persistence of pain at a specified body site. The pain descriptors can be automatically generated using the pain data such as the pain drawing provided by the subject, and quantitatively describe the pain in a pain-area level or in a subject-level.

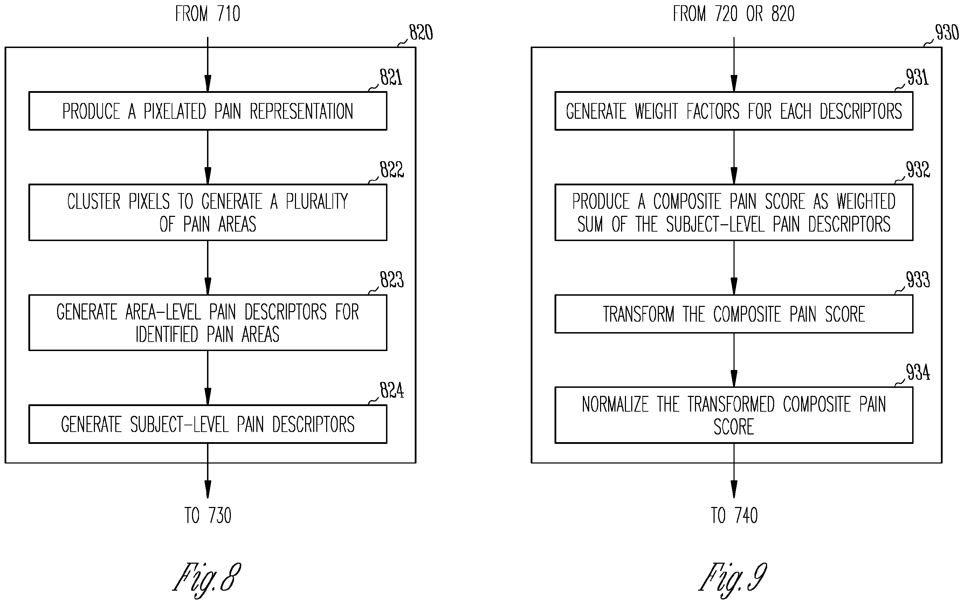

At 730, a composite pain score can be produced using the plurality of the pain descriptors. The composite pain score can quantify overall pain in the subject. The composite pain score, such as an integrated pain quantification index (IPQI), can be represented as a numerical or categorical value, and can be calculated using a linear or nonlinear combination of the pain descriptors, such as described in a method as illustrated in FIG. 9.

At 740, a presentation of information about the subject's overall pain, including the composite pain score such as the IPQI, can be produced. The information can be displayed in a monitor or other displaying devices included in an output unit. Additional information, including pain descriptors for one or more identified pain areas, or pain descriptors in a subject-level that characterize overall pain intensity, pain persistence, or anatomical extent, can also be presented to a clinician. In an example, a reference pain score before delivery of a prescribed pain therapy can be produced, and a pain therapy efficacy indicator, based on a difference between the composite pain score (such as the IPQI) and the reference pain score can be produced and presented to the clinician. The pain therapy efficacy indicator can demonstrate effectiveness of the prescribed therapy. The clinician can modify the pain therapy when the pain therapy efficacy indicator does not meet a therapeutic criterion.

FIG. 8 illustrates generally an example of a method 820 for generating pain descriptors. The method 820 can be a specific embodiment of pain descriptor generation method at step 720 of FIG. 7. The method 820 can include several steps of using the pain data, such as obtained from the step 710, to generate subject-level pain descriptors for use to produce the composite pain score at 730.

At 821, a pixelated pain representation is produced using the patient pain data. The patient pain drawing can be converted into a plurality of pixels each represented by respective pixel coordinate corresponding to anatomical point locations of the subject. Each pixel can be associated with respective pain descriptors indicating one or more of presence, intensity, and temporal pattern of pain at the corresponding pixel. The pixels are then grouped at 822 to produce N pain areas, such as by grouping the pixels having a value of "1" that indicate presence of pain at the corresponding pixel location and are separated by no more than a specified number of pixels having a value of "0" that indicate absence of pain at the corresponding pixel location. In an example, grouping can be performed using clustering analysis of the pixels associated with multi-dimensional pain descriptors. Each pain area comprises pixels with respective pain descriptors meeting a specified criterion. Examples of the clustering analysis can include a *k*-means clustering that minimizes the within-cluster sum of squares of a similarity metric between the pixels and a cluster centroid. The similarity metric can be determined using an Euclidian distance, a cosine distance or correlation coefficient, or Mahalanobis distance, among others.

At 823, pain area-level pain descriptors can be generated for each of the *N* pain areas. The area-level pain descriptors can include two or more of: a size of pain area, an average pain intensity and a peak intensity, a pain persistence level, or one or more dermatome numbers associated with the respective pain area. The size of a pain area can indicate a proportion of a body of the subject affected by the respective pain area. The average pain intensity and the peak pain intensity can each be represented by a numerical value within a specified range indicating respectively average severity and highest severity of the pain at the respective pain area within a specified period of time, such as in past 7-10 days. The pain persistence level can be represented by a numerical value within a specified range indicating frequency, duration, or continuity of the pain at the respective pain area within a specified period of time, such as in past 7-10 days. The dermatome numbers can include at least a maximum dermatome number and a minimum dermatome number. The dermatomes as covered by the respective pain area can be mapped to dermatome numbers using the mapping table as illustrated in FIG. 4.

At 824, the area-level pain descriptors for all *N* pain areas can be integrated to produce one or more subject-level pain descriptors. The subject-level pain descriptors can include one or more of: a total number of pain areas *N,* an anatomical pain span, an accumulative pain intensity, and an accumulative pain persistence level across at least some of the pain areas. The anatomical pain span can be computed as a difference between a maximum dermatome number across the *N* pain areas and a minimum dermatome number across the *N* pain areas, such as according to equation (1). The anatomical pain span thus indicates a range of dematomal regions to which the pain area extend. The accumulative pain intensity can be computed as a sum, across the *N* pain areas, of size-scaled average of the peak pain intensity and the average pain intensity of the respective pain area, such as according to equation (2). The accumulative pain persistence level can be computed as a sum, across the *N* pain areas, of size-scaled pain persistence level, such as according to equation (3). The subject-level pain descriptors can be used to produce a composite pain score at 740.

FIG. 9 illustrates generally an example of a method 930 for producing a composite pain score. The method 930 can be a specific embodiment of composite pain score generation method at step 730 of FIG. 7. The method 930 can include several steps of using at least some of the pain descriptors as obtained from the step 720, or the subject-level pain descriptors as obtained from step 820, to generate the composite pain score, such as the IPQI, for use in producing the presentation of pain information at 740.

At 931, weight factors used for linear or nonlinear combination of the subject-level pain descriptors can be generated. In an example, the weight factors can be specified by a system user. In another example, the weight factors can be determined based on principal component analysis (PCA) of a database of patient pain data. PCA can reduce a set of potentially correlated variables (*e.g.* pain distribution, pain intensity, and persistence) into a smaller set of variables that preserves most of the information. In an example, the PCA of the patient pain data can include standardizing each pain variable such as by subtracting off its mean and dividing by its standard deviation. A matrix of pain variables can be formed using the pain variables. Such matrix can then be factorized such as by using singular value decomposition (SVD). The right-singular vector, corresponding to the largest singular value, can be retained as the weight factors.

The generated weights can be used at 932 to produce a raw composite score as a linear or nonlinear combination of the subject-level pain descriptors, such as a weighted sum of the subject-level pain descriptors according to equation (4). At 933, the raw composite score can then be transformed, such as by using a logarithm transformation according to equation (5). The logarithm transformation can improve the sensitivity of the composite pain score to changes in the subject-level pain descriptors when the patient experiences more localized or moderate amount of pain.

At 934, a normalized composite pain score, integrated pain quantification index (IPQI), can be produced by normalizing the composite pain score with respect to an expected range of the composite pain score. The expected range can be determined as the difference between a theoretical maximum and a theoretical minimum. The theoretical minimum corresponds to the raw composite pain score being set to zero, and the theoretical maximum can be obtained when all subject-level pain descriptors are set to maximum value within their respective ranges. The theoretical minimum and maximum composite pain score can be determined according to equations (6) and (7), respectively. The normalized composite pain score, IPQI, such as determined according to equation (8), can take value between 0 and 1, where IPQI = 0 corresponds to no pain sensation and IPQI = 1 corresponds to pain with maximal severity.

In some examples, the method 700, or variants of any part of the method 700 such as the method 820 of generating the pain descriptors, or the method 930 of producing the composite score, can be implemented as instructions stored in a machine-readable storage medium. The machine can be in a form of a computer system, which can include a processor, memory, video display unit, an alpha-numeric input device, and a user interface with a navigation device, a disk drive unit, a signal generation device, a network interface device, among others. The instructions can cause machine to perform any part of the method 700 or any variants thereof, including, for example receiving pain data indicative of pain in a subject, and generating a plurality of pain descriptors using the pain data, where the plurality of pain descriptors can be indicative of two or more of pain intensity, spatial extent of pain, or temporal pattern of pain. The instructions can also cause the machine to produce a composite pain score quantifying overall pain using the plurality of the pain descriptors, and produce a presentation of information about the pain in the subject, including the composite pain score, for displaying in an output unit.

The machine can operate as a standalone device or may be connected (e.g., networked) to other machines. In a networked deployment, the machine may operate in the capacity of a server or a client machine in server-client network environment, or as a peer machine in a peer-to-peer (or distributed) network environment. The machine may be a personal computer (PC), a tablet PC, a set-top box (STB), a PDA, a cellular telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein.

The machine-readable medium may include a single medium or multiple media (e.g., a centralized or distributed database, and/or associated caches and servers) that store the one or more instructions or data structures. The term "machine-readable storage medium" shall also be taken to include any tangible medium that is capable of storing, encoding or carrying instructions for execution by the machine and that cause the machine to perform any one or more of the methods of the present invention, or that is capable of storing, encoding or carrying data structures used by or associated with such instructions. The term "machine-readable storage medium" shall accordingly be taken to include, but not be limited to, solid-state memories, and optical and magnetic media. Specific examples of machine-readable media include non-volatile memory, including by way of example, semiconductor memory devices (e.g., erasable programmable read-only memory (EPROM), electrically erasable programmable read-only memory (EEPROM)) and flash memory devices; magnetic disks such as internal hard disks and removable disks; magneto-optical disks; and CD-ROM and DVD-ROM disks. A "machine-readable storage medium" shall also include devices that may be interpreted as transitory, such as register memory, processor cache, and RAM, among others. The definitions provided herein of machine-readable medium and machine-readable storage medium are applicable even if the machine-readable medium is further characterized as being "non-transitory." For example, any addition of "non-transitory," such as non-transitory machine-readable storage medium, is intended to continue to encompass register memory, processor cache and RAM, among other memory devices.

In various examples, the instructions may further be transmitted or received over a communications network using a transmission medium. The instructions may be transmitted using the network interface device and any one of a number of well-known transfer protocols (e.g., HTTP). Examples of communication networks include a LAN, a WAN, the Internet, mobile telephone networks, plain old telephone (POTS) networks, and wireless data networks (e.g., WiFi and WiMax networks). The term "transmission medium" shall be taken to include any intangible medium that is capable of storing, encoding or carrying instructions for execution by the machine, and includes digital or analog communications signals or other intangible media to facilitate communication of such software.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using combinations or permutations of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

**Claims**

1. A system for assessing pain in a subject, comprising:

   a data receiver (210), configured to receive pain data indicative of pain in the subject;
   a processor circuit (220), configured to:

      generate (720) a plurality of pain descriptors using the pain data, the plurality of pain descriptors indicative of two or more of pain intensity, spatial extent of pain, or temporal pattern of pain; and
      produce (730) a composite pain score using the plurality of the pain descriptors, the composite pain score quantifying overall pain;
      wherein the processor circuit comprises an image processing circuit (501) configured to:

         produce (821) a pixelated pain representation using the received pain data, including pixelating a patient pain drawing into a plurality of pixels corresponding to anatomical point locations of the subject, each of the plurality of pixels associated with respective pain descriptors indicating one or more of presence, intensity, or temporal pattern of pain at the corresponding pixel; and
         group (822) the pixels into one or more pain areas, each of the pain areas comprising pixels with respective pain descriptors meeting a specified criterion; and
         the system comprising an output unit (230) configured to produce (740) a presentation of information about the pain in the subject including the composite pain score.

2. The system of claim 1, wherein the composite pain score is indicative of overall pain in the subject after delivery of a pain therapy, and wherein the output unit is configured to produce the presentation including a pain therapy efficacy indicator based on a difference between the composite pain score and a reference pain score indicative of overall pain in the subject prior to delivery of the pain therapy.

3. The system of any one of claims 1 or 2, wherein the data receiver is configured to receive the pain data including one or more of a patient pain drawing, a patient questionnaire, or a dermatome map.

4. The system of any one of claims 1 through 3, further comprising a user interface unit communicatively coupled to the data receiver, the user interface unit configured to enable the subject to input the pain data.

5. The system of any one of claims 1-4, wherein the processor circuit is configured to generate, for each of the pain areas, a respective plurality of pain descriptors including two or more of:

a size of pain area indicating a proportion of a body of the subject affected by the respective pain area;
an average pain intensity in the respective pain area;
a peak pain intensity in the respective pain area;
a pain persistence level in the respective pain area; or
one or more dermatome numbers indicating vertebral levels of the subject affected by the respective pain area.

6. The system of claim 5, wherein the one or more dermatome numbers include integers each mapped to the vertebral levels associated with dermatomes C2 to S5, the one or more dermatome numbers including a maximum dermatome number and a minimum dermatome number associated with the respective pain area.

7. The system of claim 5, wherein the processor circuit is further configured to generate one or more subject-level pain descriptors using the respective plurality of pain descriptors for the pain areas, the subject-level pain descriptors including one or more of:

an accumulative pain intensity across at least some of the pain areas;
an accumulative pain persistence level across at least some of the pain areas;
an anatomical pain span indicating a range of dematomal regions to which the pain areas extend; or
a total number of pain areas.

8. The system of claim 7, wherein the accumulative pain intensity is a combination, across the pain areas, of one or both of the peak pain intensity and the average pain intensity in the respective pain area weighted by the size of the respective pain area.

9. The system of any one of claims 7 or 8, wherein the accumulative pain persistence level is a combination, across the pain areas, of the pain persistence level in the respective pain area weighted by the size of the respective pain area.

10. The system of any one of claims 7 through 9, wherein the anatomical pain span is a difference between a maximum dermatome number across the pain areas and a minimum dermatome number across the pain areas.

11. The system of any one of claims 7 through 10, wherein the processor circuit is configured to produce the composite pain score using a linear or nonlinear combination of the subject-level pain descriptors.

12. The system of claim 11, wherein the processor circuit is configured to produce the composite pain score using a weighted sum of the subject-level pain descriptors.

13. The system of any one of claims 11 or 12, wherein the processor circuit is configured to perform a transformation of the composite pain score.

14. The system of any one of claims 11 through 13, where in the processor circuit is further configured to produce a normalized composite pain score by normalizing the composite pain score with respect to an expected range of the composite pain score.

**Patentansprüche**

1. System zur Schmerzbeurteilung bei einem Patienten, aufweisend:

einen Datenempfänger (210), der zum Empfangen von Schmerzdaten konfiguriert ist, die die Schmerzen des Patienten angeben;
eine Prozessorschaltung (220), die konfiguriert ist zum:

Generieren (720) von mehreren Schmerzdeskriptoren mit den Schmerzdaten, wobei die mehreren Schmerzdeskriptoren mindestens zwei der Faktoren Schmerzintensität, räumliche Ausdehnung des Schmerzes oder zeitlicher Verlauf des Schmerzes angeben; und
Erstellen (730) eines zusammengesetzten Schmerzwerts mit den mehreren Schmerzdeskriptoren, wobei der zusammengesetzte Schmerzwert die Gesamtschmerzen quantifiziert;

wobei die Prozessorschaltung eine Bildverarbeitungsschaltung (501) aufweist, die konfiguriert ist zum:

Erstellen (821) einer Schmerzdarstellung auf Pixelbasis mit den empfangenen Schmerzdaten, darunter Pixeln einer Patientenschmerzzeichnung in mehrere Pixel, die anatomischen Punktpositionen des Patienten entsprechen, wobei jedes der mehreren Pixel mit jeweiligen Schmerzdeskriptoren assoziiert ist, die Vorhandensein und/oder Intensität und/oder zeitlichen Verlauf des Schmerzes am entsprechenden Pixel angeben; und

Gruppieren (822) der Pixel in einen oder mehrere Schmerzbereiche, wobei jeder der Schmerzbereiche Pixel mit entsprechenden Schmerzdeskriptoren aufweisen, die ein angegebenes Kriterium erfüllen; und das System eine Ausgabeeinheit (230) aufweist, die zum Erstellen (740) einer Präsentation der Informationen über die Schmerzen beim Patienten einschließlich des zusammengesetzten Schmerzwerts konfiguriert ist.

2. System nach Anspruch 1, wobei der zusammengesetzte Schmerzwert die Gesamtschmerzen des Patienten nach Verabreichung einer Schmerztherapie angibt und wobei die Ausgabeeinheit konfiguriert ist, die Präsentation einschließlich eines Schmerztherapie-Wirksamkeitsindikators zu erstellen, der auf einer Differenz zwischen dem zusammengesetzten Schmerzwert und einem Schmerzbezugswert, der die Gesamtschmerzen des Patienten vor Verabreichung der Schmerztherapie angibt, basiert.

3. System nach einem der Ansprüche 1 oder 2, wobei der Datenempfänger zum Empfangen der Schmerzdaten einschließlich einer Patientenschmerzzeichnung und/oder eines Patientenfragebogens und/oder einer Dermatomkarte konfiguriert ist.

4. System nach einem der Ansprüche 1 bis 3, ferner aufweisend eine Benutzerschnittstelleneinheit, die kommunikativ mit dem Datenempfänger gekoppelt ist, wobei die Benutzerschnittstelleneinheit konfiguriert ist, dem Patienten das Eingeben der Schmerzdaten zu ermöglichen.

5. System nach einem der Ansprüche 1-4, wobei die Prozessorschaltung konfiguriert ist, für jeden der Schmerzbereiche entsprechend mehrere Schmerzdeskriptoren zu erstellen, darunter mindestens zwei der folgenden:

eine Größe des Schmerzbereichs, die einen vom jeweiligen Schmerzbereich betroffenen Anteil des Körpers des Patienten angibt;
eine durchschnittliche Schmerzintensität im jeweiligen Schmerzbereich;
eine Spitzenschmerzintensität im jeweiligen Schmerzbereich;
ein Schmerzpersistenzniveau im jeweiligen Schmerzbereich; oder
eine oder mehrere Dermatomzahlen, die vom jeweiligen Schmerzbereich betroffene Wirbelebenen des Patienten angeben.

6. System nach Anspruch 5, wobei die eine oder die mehreren Dermatomzahlen ganze Zahlen umfassen, die den mit den Dermatomen C2 bis S5 assoziierten Wirbelebenen zugeordnet sind, wobei die eine oder die mehreren Dermatomzahlen eine mit dem jeweiligen Schmerzbereich assoziierte größte und kleinste Dermatomzahl umfassen.

7. System nach Anspruch 5, wobei die Prozessorschaltung ferner zum Erzeugen eines oder mehrerer patientenseitiger Schmerzdeskriptoren mit den jeweiligen mehreren Schmerzdeskriptoren für die Schmerzbereiche konfiguriert ist, wobei die patientenseitigen Schmerzdeskriptoren einen oder mehrere der folgenden umfassen:

eine kumulative Schmerzintensität über mindestens einige der Schmerzbereiche;
ein kumulatives Schmerzpersistenzniveau über mindestens einige der Schmerzbereiche;
eine anatomische Schmerzspanne, die einen Bereich der Dermatomregionen angibt, auf den sich die Schmerzbereiche erstrecken oder eine Gesamtanzahl von Schmerzbereichen.

8. System nach Anspruch 7, wobei die kumulative Schmerzintensität über die Schmerzbereiche hinweg eine Kombination der Spitzenschmerzintensität und/oder der durchschnittlichen Schmerzintensität im jeweiligen Schmerzbereich, gewichtet nach der Größe des jeweiligen Schmerzbereichs darstellt.

9. System nach einem der Ansprüche 7 oder 8, wobei das kumulative Schmerzpersistenzniveau über die Schmerzbereiche hinweg eine Kombination des Schmerzpersistenzniveaus im jeweiligen Schmerzbereich, gewichtet nach der Größe des jeweiligen Schmerzbereichs darstellt.

**10.** System nach einem der Ansprüche 7 bis 9, wobei die anatomische Schmerzspanne eine Differenz zwischen einer größten Dermatomzahl über die Schmerzbereiche hinweg und einer kleinsten Dermatomzahl über die Schmerzbereiche hinweg darstellt.

**11.** System nach einem der Ansprüche 7 bis 10, wobei die Prozessorschaltung zum Erstellen des zusammengesetzten Schmerzwerts mit einer linearen oder nichtlinearen Kombination der patientenseitigen Schmerzdeskriptoren konfiguriert ist.

**12.** System nach Anspruch 11, wobei die Prozessorschaltung zum Erstellen des zusammengesetzten Schmerzwerts mit einer gewichteten Summe der patientenseitigen Schmerzdeskriptoren konfiguriert ist.

**13.** System nach einem der Ansprüche 11 oder 12, wobei die Prozessorschaltung zum Durchführen einer Transformation des zusammengesetzten Schmerzwerts konfiguriert ist.

**14.** System nach einem der Ansprüche 11 bis 13, wobei die Prozessorschaltung ferner zum Erstellen eines normalisierten zusammengesetzten Schmerzwerts durch Normalisieren des zusammengesetzten Schmerzwerts in Bezug auf einen erwarteten Bereich des zusammengesetzten Schmerzwerts konfiguriert ist.

**Revendications**

**1.** Système d'évaluation de la douleur chez un sujet, comprenant :

un récepteur de données (210), configuré de manière à recevoir des données de douleur indiquant une douleur chez le sujet ;
un circuit de processeur (220), configuré de manière à :

générer (720) une pluralité de descripteurs de douleur en utilisant les données de douleur, la pluralité de descripteurs de douleur indiquant deux caractéristiques ou plus parmi l'intensité de la douleur, l'étendue spatiale de la douleur, ou un modèle temporel de la douleur ; et
produire (730) un score de douleur composite en utilisant la pluralité des descripteurs de douleur, le score de douleur composite quantifiant la douleur globale ;

dans lequel le circuit de processeur comprend un circuit de traitement d'image (501) configuré de manière à :

produire (821) une représentation de douleur pixélisée en utilisant les données de douleur reçues, et notamment pixeliser un dessin de douleur de patient en une pluralité de pixels correspondant à des emplacements de points anatomiques du sujet, chaque pixel de la pluralité de pixels étant associé à des descripteurs de douleur respectifs indiquant un ou plusieurs éléments parmi la présence, l'intensité ou le modèle temporel de la douleur au niveau du pixel correspondant ; et
regrouper (822) les pixels en une ou plusieurs zones douloureuses, chacune des zones douloureuses comprenant des pixels avec des descripteurs de douleur respectifs satisfaisant un critère spécifié ; et

le système comprenant une unité de sortie (230) configurée de manière à produire (740) une présentation d'informations concernant la douleur chez le sujet, y compris le score de douleur composite.

**2.** Système selon la revendication 1, dans lequel le score de douleur composite est indicatif de la douleur globale chez le sujet après l'administration d'un traitement thérapeutique antidouleur, et dans lequel l'unité de sortie est configurée de manière à produire la présentation, y compris un indicateur d'efficacité de traitement thérapeutique antidouleur, sur la base d'une différence entre le score de douleur composite et un score de douleur de référence indicatif de la douleur globale chez le sujet avant l'administration du traitement thérapeutique antidouleur.

**3.** Système selon l'une quelconque des revendications 1 et 2, dans lequel le récepteur de données est configuré de manière à recevoir les données de douleur, y compris un ou plusieurs éléments parmi un dessin de douleur de patient, un questionnaire patient ou une carte des dermatomes.

**4.** Système selon l'une quelconque des revendications 1 à 3, comprenant en outre une unité d'interface utilisateur couplée en communication au récepteur de données, l'unité d'interface utilisateur étant configurée de manière à permettre au sujet de saisir des données de douleur.

5. Système selon l'une quelconque des revendications 1 à 4, dans lequel le circuit de processeur est configuré de manière à générer, pour chacune des zones douloureuses, une pluralité respective de descripteurs de douleur incluant deux des éléments suivants ou plus :

une taille de zone douloureuse indiquant une proportion d'un corps du sujet affecté par la zone douloureuse respective ;
une intensité moyenne de la douleur dans la zone douloureuse respective ;
une intensité maximale de la douleur dans la zone douloureuse respective ;
un niveau de persistance de la douleur dans la zone douloureuse respective ; ou
un ou plusieurs nombres de dermatomes indiquant des niveaux vertébraux du sujet affectés par la zone douloureuse respective.

6. Système selon la revendication 5, dans lequel ledit un ou lesdits plusieurs nombres de dermatomes incluent des nombres entiers qui sont chacun mis en correspondance avec les niveaux vertébraux associés aux dermatomes C2 à S5, ledit un ou lesdits plusieurs nombres de dermatomes incluant un nombre de dermatomes maximum et un nombre de dermatomes minimum associés à la zone douloureuse respective.

7. Système selon la revendication 5, dans lequel le circuit de processeur est en outre configuré de manière à générer un ou plusieurs descripteurs de douleur au niveau du sujet en utilisant la pluralité respective de descripteurs de douleur pour les zones douloureuses, les descripteurs de douleur au niveau du sujet incluant un ou plusieurs des éléments suivants :

une intensité de douleur cumulée à travers au moins certaines des zones douloureuses ;
un niveau de persistance de douleur cumulé à travers au moins certaines des zones douloureuses ;
une étendue de douleur anatomique indiquant une plage de régions dématomiques auxquelles les zones douloureuses s'étendent ; ou
un nombre total de zones douloureuses.

8. Système selon la revendication 7, dans lequel l'intensité de douleur cumulée correspond à une combinaison, à travers les zones douloureuses, de l'une ou des deux intensités parmi l'intensité maximale de la douleur et l'intensité moyenne de la douleur dans la zone douloureuse respective, pondérée par la taille de la zone douloureuse respective.

9. Système selon l'une quelconque des revendications 7 et 8, dans lequel le niveau de persistance de douleur cumulé correspond à une combinaison, à travers les zones douloureuses, du niveau de persistance de la douleur dans la zone douloureuse respective, pondéré par la taille de la zone douloureuse respective.

10. Système selon l'une quelconque des revendications 7 à 9, dans lequel l'étendue de douleur anatomique correspond à une différence entre un nombre de dermatomes maximum dans les zones douloureuses et un nombre de dermatomes minimum dans les zones douloureuses.

11. Système selon l'une quelconque des revendications 7 à 10, dans lequel le circuit de processeur est configuré de manière à produire le score de douleur composite en utilisant une combinaison linéaire ou non linéaire des descripteurs de douleur au niveau du sujet.

12. Système selon la revendication 11, dans lequel le circuit de processeur est configuré de manière à produire le score de douleur composite en utilisant une somme pondérée des descripteurs de douleur au niveau du sujet.

13. Système selon l'une quelconque des revendications 11 et 12, dans lequel le circuit de processeur est configuré de manière à mettre en oeuvre une transformation du score de douleur composite.

14. Système selon l'une quelconque des revendications 11 à 13, dans lequel le circuit de processeur est en outre configuré de manière à produire un score de douleur composite normalisé, en normalisant le score de douleur composite par rapport à une plage attendue du score de douleur composite.

*Fig. 1*

_/—200_

| CONTROLLER CIRCUIT | 250 |
| USER INTERFACE UNIT | 240 |

DATA RECEIVER ~210

PROCESSOR CIRCUIT ~220

PAIN DESCRIPTOR GENERATOR ~222

COMPOSITE SCORE GENERATOR ~224

OUTPUT UNIT ~230

_Fig.2_

Fig. 3

| DERMATOME | DERMATOME NUMBER |
|:---:|:---:|
| C2 | 1 |
| C3 | 2 |
| ⋮ | ⋮ |
| C8 | 7 |
| T1 | 8 |
| T2 | 9 |
| ⋮ | ⋮ |
| T12 | 19 |
| L1 | 20 |
| L2 | 21 |
| ⋮ | ⋮ |
| L5 | 24 |
| S1 | 25 |
| S2 | 26 |
| ⋮ | ⋮ |
| S5 | 29 |

*Fig. 4*

Fig. 5

*Fig.6*

EP 3 302 236 B1

700

```
                                                    ⌐710
        ┌─────────────────────────────────────────────┐
        │ RECEIVE PAIN DATA INDICATIVE OF MULTI-SITE PAIN │
        └─────────────────────────────────────────────┘
                              │
                              ▼                     ⌐720
        ┌─────────────────────────────────────────────┐
        │           GENERATE PAIN DESCRIPTORS           │
        └─────────────────────────────────────────────┘
                              │
                              ▼                     ⌐730
        ┌─────────────────────────────────────────────┐
        │          PRODUCE A COMPOSITE PAIN SCORE        │
        └─────────────────────────────────────────────┘
                              │
                              ▼                     ⌐740
        ┌─────────────────────────────────────────────┐
        │      PRODUCE A PRESENTATION OF INFORMATION     │
        │             ABOUT THE PAIN QUALITY             │
        └─────────────────────────────────────────────┘
```

*Fig. 7*

FROM 710

```
820
 821  PRODUCE A PIXELATED PAIN REPRESENTATION

 822  CLUSTER PIXELS TO GENERATE A PLURALITY
       OF PAIN AREAS

 823  GENERATE AREA-LEVEL PAIN DESCRIPTORS FOR
       IDENTIFIED PAIN AREAS

 824  GENERATE SUBJECT-LEVEL PAIN DESCRIPTORS
```

TO 730

*Fig.8*

FROM 720 OR 820

```
930
 931  GENERATE WEIGHT FACTORS FOR EACH DESCRIPTORS

 932  PRODUCE A COMPOSITE PAIN SCORE AS WEIGHTED
       SUM OF THE SUBJECT-LEVEL PAIN DESCRIPTORS

 933  TRANSFORM THE COMPOSITE PAIN SCORE

 934  NORMALIZE THE TRANSFORMED COMPOSITE PAIN
       SCORE
```

TO 740

*Fig.9*

EP 3 302 236 B1

**EP 3 302 236 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62170246
- US 2001037222 A1
- WO 2015036661 A1
- US 2013018275 A1